# EUROPEAN PATENT SPECIFICATION

(11) **EP 1 283 170 B1**
(45) Date of publication and mention of the grant of the patent: **30.08.2006**
(21) Application number: 02015735.0
(22) Date of filing: 13.07.2002
(51) Int. Cl.: B65B 9/18, B65B 67/12, A61L 11/00, B09B 3/00

(54) **Cassette of foil**
Folienkassette
Cassette à feuille

(30) Priority: 10.08.2001 SE 0102691
(43) Date of publication of application: 12.02.2003
(73) Proprietor: Paxxo AB, 202 11 Malmö (SE)
(72) Inventor: Nordström, Peter, 713 31 Nora (SE)
(74) Representative: Karlsson, Leif Gunnar Börje

(56) References cited:
- WO-A-97/18992
- DE-A- 2 239 880
- FR-A- 2 798 361
- US-A- 5 899 049

## Description

### Technical Field

The present invention concerns a waste handling device for safe disposal of hazardous medical waste or more specifically a cassette of foil to be used in said device. Even though the device is developed for use within the medical field, a person skilled in the art realises that it may also be used for other types of hazardous waste.

### Prior Art

In the devices for safe disposal of hazardous medical waste used at present it is common that the foil is delivered in folded form in a ring, this is often referred to as a cassette. Such a device is disclosed in WO 97 18992, for example. In the description and claims below the expression "cassette" or "cassette of foil" is used to signify the complete unit to be placed in the device. Thus, the cassette may include parts in addition to the foil. The cassette is to be placed in the device with the ring folded in a prescribed way. The purpose of the foil is that it should be feed a short distance and heat-sealed to enclose the medical waste as soon as the waste has been discarded.

Many users have found it difficult to install the cassette in the prescribed way when placing it in the device. For different reasons people often do not take time to read manuals or the like before installing a new cassette. If the foil is placed incorrectly there is a risk of malfunction of the device.

In view of the above there is a need for a cassette that is easy to apply to the device, reducing the risk of erroneous handling of the device.

### The Invention

Thus, one object of the present invention is to furnish a cassette that is easy to handle and place in the device for safe disposal of hazardous medical waste.

This object is met by a cassette of foil to be placed in a device for safe disposal of waste. The cassette is prefolded into a form making it easy to install in the device, as set out in the independent claim.

The device normally comprises a housing having a central through opening, feeding means, heat sealing means and a collection box.

As stated above, the previously known cassettes are often found hard to install and often only people having been shown how to do it will manage it. The new cassette together with instructions printed directly on the cassette makes it possible for anyone to install cassettes of foil.

The cassette may be installed swiftly and the safety of the device will increase as the risk of erroneous handling is decreased.

### Brief Description of the Drawings

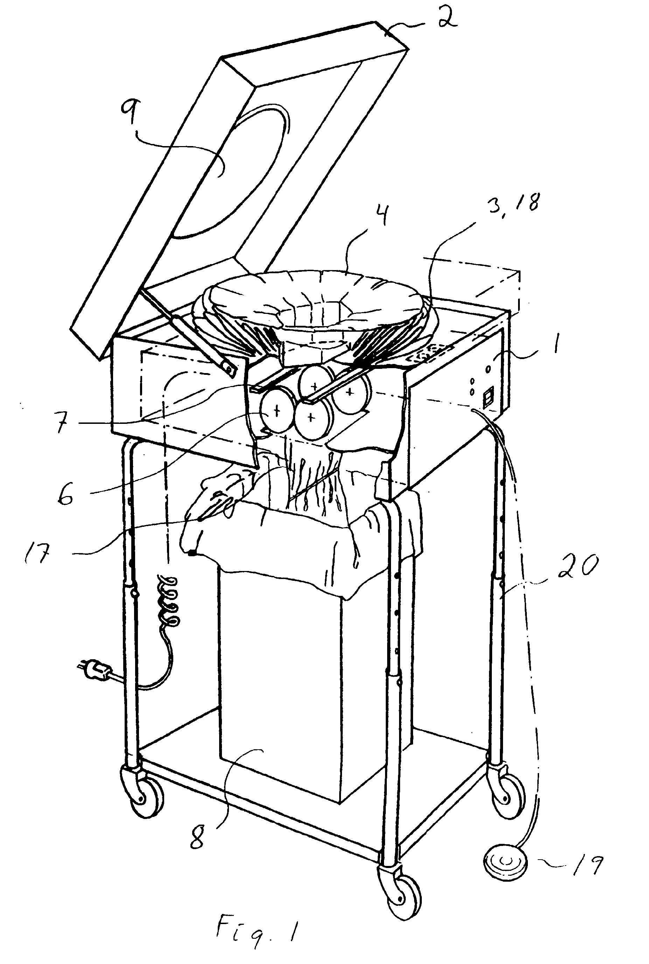

Fig. 1 is a perspective view, partly broken, of a device for handling hazardous waste.
Fig. 2 is a plan view of a part of the device of Fig. 1.
Fig. 3 is a cross-section view taken along the line III-III of Fig. 2 and with parts added.
Fig. 4 is a perspective view partly in section of a previously used cassette.
Fig. 5 is a perspective view of the cassette of Fig. 4 folded into the form it is to have when installed in the device.
Figs. 6 to 8 are perspective view of one example of a cassette according to the present invention shown in different positions.

### Detailed Description of Preferred Embodiments

As the present invention concerns an improvement of an existing device, we will first describe said existing device. A person skilled in the art realises that it is possible to use the present invention with other similar devices designed differently.

The device is built around a housing or box 1 to which a cover 2 is connected by means of one or more hinges or the like. Preferably some kind of locking means is arranged to be able to hold the cover 2 in an open position. In the shown embodiment the housing is placed on a stand 20 having wheels. A person skilled in the art realises that many types of stands may be used with or without wheels. But as it is of no importance for the present invention as such it will not be described further here.

The housing 1 has a central through opening 10, furnished with an upwardly projecting edge 5. The projecting edge 5 is to receive a funnel 4 and is also hindering that the cassette by mistake will be placed over any part of the opening 10. The opening 10 of the housing 1 and an opening 9 of the cover 2 are formed to be in line when the cover 2 is placed on top of the housing 1.

According to the previous technique a cassette 18 including a foil 17 is placed surrounding the projecting edge 5 of the housing 1. The cassette 18 has the form of a folded tube. A funnel 4 is positioned on the projecting edge 5 in such a way that the cassette 18 is placed between the funnel 4 and the housing 1. One end of the foil 17 is drawn from the cassette 18 around the outside of the funnel 4 and through the centre of the funnel 4 and down through the opening 10 of the housing 1. The foil 17 is feed downwards by means of a feeding device 6, which in the shown embodiment is formed by pairs of wheels. The tube is heat-sealed by means of a welding or heat-sealing device 7. The feeding device 6 and the heat-sealing device 7 are placed in the lower part of the housing 1, under the through opening 10.

The feeding and heat-sealing of the foil 3 is done semi-automatically, i.e. the feeding and heat-sealing cycle is started by a user but is then performed automatically. In the shown embodiment the feeding cycle is started in that a pedal 19 is activated. A person skilled in the art realises that any suitable operating device may be used, such as a button, a handle etc. As long as the pedal 19 is activated the foil 17 will be fed downwards. When the pedal 19 is no longer activated the heat-sealing device 7 will go into contact with the foil 17. After the heat-sealing the foil will be feed downwards a predetermined distance to form a pouch to receive waste. By the heat-sealing a number of airtight bags are produced containing the waste material. Said bags are delivered to a collection box 8 placed under the housing 1. When a new foil 17 is inserted it must be heat-sealed before it starts to receive waste material.

In use waste material, preferably hazardous medical waste, is placed into the funnel 4 lined with the foil 17. Then a pedal or the like is activated, whereby the foil 17 is feed downwards past the jaws of the heat-sealing device 7 by means of the feeding device 6. Thereafter the foil 17 is heat-sealed in that the jaws of the heat-sealing device 7 will go into contact with the foil 17. Hereby, the hazardous medical waste is placed in a hermetically heat-sealed bag formed of the foil and the device may receive a new batch of hazardous waste. As stated above, after the heat-sealing the foil 17 will be feed downwards to form a pouch for receiving the new batch of waste.

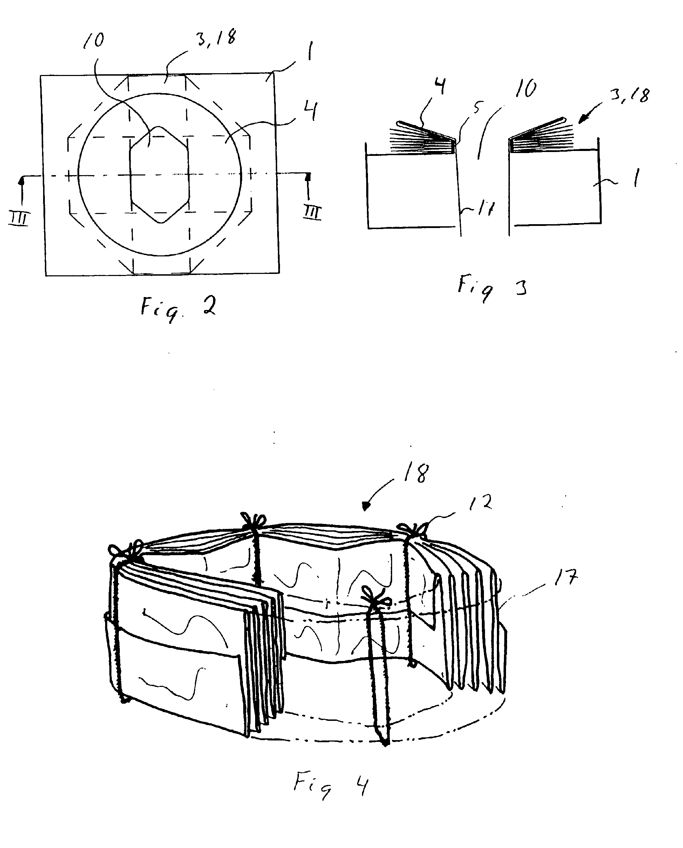

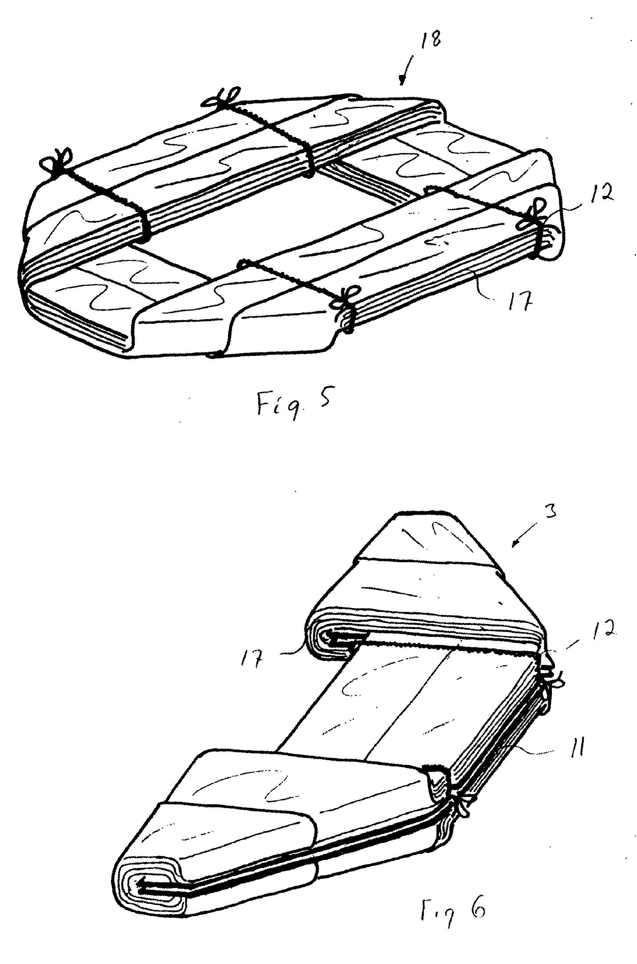

Even though the previously known device works well, in some instances there have been problems regarding placing of the cassette 18 in a correct way in the housing 1 and to pull out the foil 17. Today the cassette 18 of the foil 17 is delivered folded in the form of a flat ring, which ring is kept together by means of four strings 12 or the like. When the cassette 18 is placed in the housing 1 it has to be folded in a specified way, as indicated in Figs. 4 and 5. The specified way is that the cassette 18 in the final inserted mode should have an octagonal, flat form. It could be said that in the octagonal form two opposing sides of the cassette 18 have been folded in over two other sides of the cassette 18. When the cassette 18 has been placed in the prescribed way the strings 12 are taken away and one end of the foil 17 is placed in the funnel 4 as described above. Many users have found it difficult to fold the cassette 18 in the prescribed way and to pull out the foil 17 when placing it in the housing 1. Thus, the present invention is developed to make it easier to place the cassette in the correct, folded way in the housing 1.

According to the invention the cassette 3 is delivered prefolded in the way it is to be placed in the housing 1, as set out in the independent claim.

The cassette 3 includes a foil 17 and one or more boards 11. The boards 11 will stabilise the cassette 3 and are each furnished with notches 14 to receive strings 12 knotted around the boards 11 and the foil 17. Furthermore, the boards 11 may be furnished with prints 13. The prints 13 may e.g. include instructions of how the cassette 3 is to be placed in the housing 1. The boards 11 are preferably made of corrugated fibreboard, but a person skilled in the art realises that many different materials may be used, such as different types of paper, plastics etc.

To facilitate installing of the cassettes 3,they may be folded along a centre line 16 as indicated in Figs. 6 to 8. Thus, the cassette 3 will be formed like a "C".

As indicated above the number of boards 11 may vary. In a first embodiment there are two boards 11, which are placed facing each other between the foil 17 when the cassette 3 is folded into the C-form. Thus, the boards 11 are placed in the middle of the "C". The cassette 3 is first inserted in the housing 1 in the C-form. It is then spread out to surround the opening 10 of the housing. In this position possible prints 13 of the boards may be viewed. The prints 13 preferably include instructions regarding the insertion of the cassette 3 in the housing 1, how it is to be spread out around the opening 10 of the housing 1 and that the strings 12 and boards 11 are to be taken away. When the cassette 3 is spread out it will have the same octagonal form as the previous cassette 18, when the previous cassette 3 has been installed correctly into the housing 1.

In other embodiments the cassette 3 will include one, two, three or four boards 11. The boards 11 may be placed on any side of the foil 17, but preferably they are placed in such a way that prints 13 may be used to show instructions for installation. Thus, in one embodiment two boards 11 are placed directed outwardly when the cassette 3 is folded to the C-form. In another embodiment one board 11 is placed directed outwardly, while one board 11 is placed in the middle when the cassette 3 is folded into the C-form. In the examples where only one board 11 is used, it is preferably placed directed outwardly when the cassette 3 is folded into the C-form. However, a person skilled in the art realises that the invention is applicable for one to four boards 11 placed in any position. The main purpose of the boards 11 is to stabilise the cassette 3.

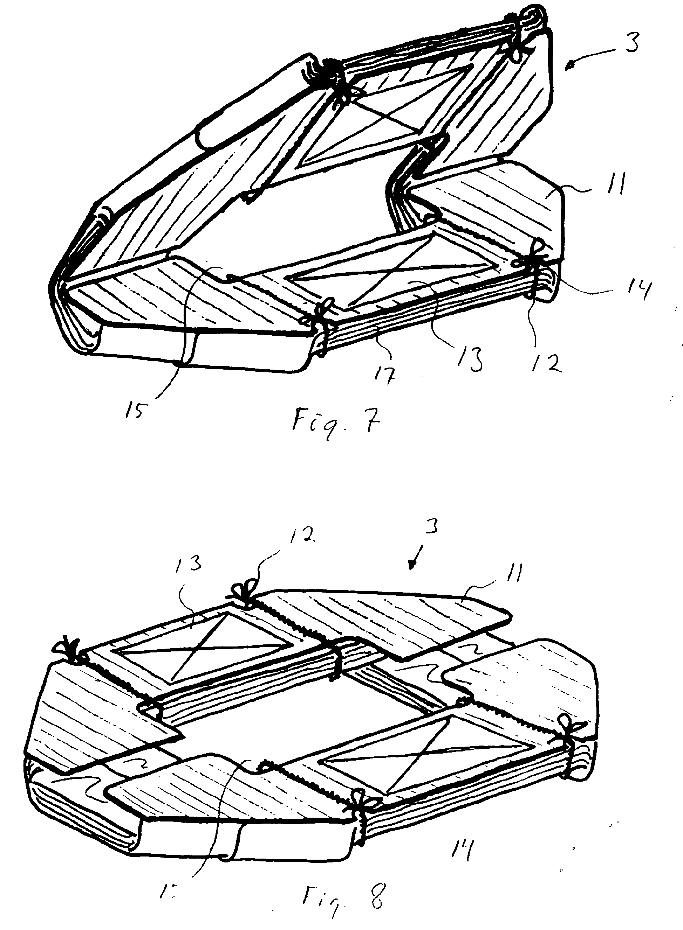

When a new cassette 3 is to be inserted the cover 2 is opened and the funnel 4 is lifted. Then the new cassette of foil 3 is normally placed in its folded C-form on one side of the opening 10 of the housing 1. The cassette 3 is then spread out to surround the opening 10 of the housing 1. The different positions for the cassette 3 as it is installed in the housing is shown in Figs. 6 to 8. The cassette is laid down in the housing 1 in the C-form as shown in Fig. 6. The boards 11 have recesses 15 that will fit around the projecting edge 5 of the housing 1. As indicated in Fig. 7 the cassette 3 is then spread out to finally reach the octagonal form as shown in Fig. 8. The next step is to lose the knots of the strings 12 and take away the strings 12 and boards 11. For embodiments having one or more boards 11 placed on the lower side of the cassette 3 when inserted in the device, the boards 11 may either be taken away directly or they may be left and taken away first before a new cassette 3 is to be installed. Then the funnel 4 is again placed around the opening 10 of the housing 1 at the same time as the end of the foil 17 is to be drawn over and into the centre of the funnel 4 as described above. Finally the cover 2 is brought down over the frame 1 and the foil 17 is feed downwards and the end heat-sealed. The device is now ready for use.

The cassette 3 does not have to be inserted in the housing 1 folded into a C-form. It is also possible to insert the cassette 3 directly in the octagonal form around the opening 10 of the housing 1. In this case the cassettes 3 are delivered prefolded into the octagonal form it is to have when installed in the housing 1, i.e. without having been folded to the C-form first.

As a further alternative the cassette 3 has no boards but is prefolded into the desired form. In this case the cassette 3 is either delivered and inserted in an octagonal form or is delivered and inserted in a C-form, which is spread out after the insertion. To assist in keeping the cassette 3 in the desired folded form, either in the C-form or the octagonal form, a wrapping of paper (not shown) may be placed around a part of the cassette. It is also possible to use other means such as clips, glue, welding etc. to keep the prefolded form of the cassette 3.

In the cassettes 3, 18 shown in the enclosed drawings only a limited number of folds are shown for convenience. A person skilled in the art realises that the actual number of folds is substantial and thus, that the total length of the foil of one cassette is also substantial.

## Claims

1. A cassette (3) of foil (17) in the form of a folded tube to be placed in a device for safe disposal of waste, **characterized in that** the cassette (3) is prefolded into an octagonal form, with two opposing sides of the cassette (3) being folded over two other sides of the cassette (3) and that the cassette (3) comprises one or more boards (11) to stabilise the cassette before installing.

2. The cassette (3) of claim 1, **characterized in that** the cassette (3) is delivered folded into a C-form along a centre line (16) and that the cassette (3) is to be inserted in the device in said C-form and then spread out surrounding a central opening (10) of the housing (1).

3. The cassette (3) of claim 1 or 2, **characterized in that** the boards (11) have notches (14) for receiving two or more strings (12) and/or that the boards (11) have a form for co-operation with a projecting edge (5) surrounding the opening (10) of the frame (1).

4. The cassette (3) of any of the previous claims, **characterized in that** the boards (11) are made of corrugated fibreboard, paper or plastics and/or that the boards (11) are furnished with prints (13).

5. The cassette (3) of any of the previous claims, **characterized in that** the one or more boards (11) are placed on the upper side, the lower side or on the inside between the foil (17) when the cassette (3) is folded into the C-form.

6. The cassette (3) of any of the previous claims, **characterized in that** at least one board (11) is directed outwards when the cassette (3) is folded into the C-form.

7. The cassette (3) of any of the previous claims, **characterized in that** at least one board (11) is directed upwards when the cassette (3) has been placed in the housing (1) and is spread out around the opening (10) of the housing (1) .

8. The cassette (3) of any of the previous claims, **characterized in that** a wrapping of paper, clips, glue or welding is used to keep the cassette (3) in the desired folded form.

## Patentansprüche

1. Kassette (3) aus Folie (17) in der Form eines gefalteten Schlauchs zum Einsetzen in eine Vorrichtung zum sicheren Entsorgen von Abfall, **dadurch gekennzeichnet, dass** die Kassette (3) in eine octagonale Form vorgefaltet ist, wobei zwei gegenüberliegende Seiten der Kassette (3) über zwei andere Seiten der Kassette (3) gefaltet sind, und dass die Kassette (3) eine oder mehrere Platten (11) zur Stabilisierung der Kassette vor dem Installieren aufweist.

2. Kassette (3) nach Anspruch 1, **dadurch gekennzeichnet, dass** die Kassette (3) in einer C-Form entlang einer zentralen Linie (16) gefaltet geliefert wird und dass die Kassette (3) in die Vorrichtung in der C-Form eingesetzt und dann ausgebreitet werden soll, wobei sie eine zentrale Öffnung (10) des Gehäuses (1) umschließt.

3. Kassette (3) nach Anspruch 1 oder 2, **dadurch gekennzeichnet, dass** die Platten (11) Einkerbungen (14) für die Aufnahme von zwei oder mehr Schnüren (12) aufweisen und/oder dass die Platten (11) eine Form zur Kooperation mit einem vorstehenden Rand (5), der die Öffnung (10) des Rahmens (1) umgibt, aufweisen.

4. Kassette (3) nach einem der vorhergehenden Ansprüche, **dadurch gekennzeichnet, dass** die Platten (11) aus Faserwellpappe, Papier oder Plastik bestehen und/oder dass die Platten (11) mit Drucken (13) versehen sind.

5. Kassette (3) nach einem der vorhergehenden Ansprüche, **dadurch gekennzeichnet, dass** eine oder mehrere der Platten (11) an der Oberseite, der Unterseite oder an der Innenseite zwischen der Folie (17) angeordnet sind, wenn die Kassette (3) in die C-Form gefaltet ist.

6. Kassette (3) nach einem der vorhergehenden Ansprüche, **dadurch gekennzeichnet, dass** zumindest eine Platte (11) nach außen gerichtet ist, wenn die Kassette (3) in die C-Form gefaltet ist.

7. Kassette (3) nach einem der vorhergehenden Ansprüche, **dadurch gekennzeichnet, dass** zumindest eine Platte (11) nach oben gerichtet ist, wenn die Kassette (3) in das Gehäuse (1) eingelegt und um die Öffnung (10) des Gehäuse (1) ausgebreitet worden ist.

8. Kassette (3) nach einem der vorhergehenden Ansprüche, **dadurch gekennzeichnet, dass** eine Verpackung aus Papier, Klemmen, Kleber oder Schweißung verwendet wird, um die Kassette (3) in der vorgesehenen gefalteten Form zu halten.

## Revendications

1. Recharge (3) de film (17) se présentant sous la forme d'un tube plié, devant être placé dans un dispositif permettant la mise au rebut de déchets en toute sécurité, **caractérisée en ce que** la recharge (3) est prépliée en une forme octogonale, deux côtés opposés de la recharge (3) étant pliés sur deux autres côtés de la recharge (3) et **en ce que** la recharge (3) comprend un ou plusieurs volets (11) pour stabiliser la recharge avant l'installation.

2. Recharge (3) selon la revendication 1, **caractérisée en ce que** la recharge (3) est fournie pliée en forme de C le long d'une ligne centrale (16) et **en ce que** la recharge (3) doit être insérée dans le dispositif selon ladite forme de C et dépliée pour entourer une ouverture centrale (10) du coffrage (1).

3. Recharge (3) selon la revendication 1 ou 2, **caractérisée en ce que** les volets (11) présentent des encoches (14) permettant de recevoir deux ou plusieurs ficelles (12) et/ou en ce que les volets (11) présentent une forme permettant une coopération avec un bord faisant saillie (5) entourant l'ouverture (10) du coffrage (1).

4. Recharge (3) selon l'une quelconque des revendications précédentes, **caractérisée en ce que** les volets (11) sont composés de carton ondulé, de papier ou de plastique et/ou en ce que les volets (11) sont dotés d'éléments imprimés (13).

5. Recharge (3) selon l'une quelconque des revendications précédentes, **caractérisée en ce que** le ou les volets (11) sont placés sur le côté supérieur, le côté inférieur ou sur l'intérieur situé entre les deux parties du film (17) lorsque la recharge (3) est pliée en forme de C.

6. Recharge (3) selon l'une quelconque des revendications précédentes, **caractérisée en ce qu'**au moins un volet (11) est dirigé vers l'extérieur lorsque la recharge (3) est pliée en forme de C.

7. Recharge (3) selon l'une quelconque des revendications précédentes, **caractérisée en ce qu'**au moins un volet (11) est dirigé vers le haut lorsque la recharge (3) a été placée dans le coffrage (1) et est dépliée autour de l'ouverture (10) du coffrage (1).

8. Recharge (3) selon l'une quelconque des revendications précédentes, **caractérisée en ce qu'**un enroulement de papier, des agrafes, de la colle ou une soudure est utilisé pour conserver la forme souhaitée de la recharge (3).
